# EUROPEAN PATENT APPLICATION

(11) **EP 2 138 166 A1**
(43) Date of publication of application: **30.12.2009**
(21) Application number: 08752091.2
(22) Date of filing: 25.04.2008
(51) Int. Cl.: A61K 9/42, A61K 47/02, A61K 47/04, A61K 47/10, A61K 47/12, A61K 47/26, A61K 47/32, A61K 47/34, A61K 47/38, A61K 47/44

(54) **PROCESS FOR PRODUCTION OF TABLET**

(30) Priority: 26.04.2007 JP 2007117475
(71) Applicant: Eisai R&D Management Co., Ltd., Tokyo 112-8088 (JP)
(72) Inventor: ONO, Nao, Honjo-shi Saitama 367-0048 (JP); SHIMOGAKI, Norio, Honjo-shi Saitama 367-0048 (JP)
(74) Representative: HOFFMANN EITLE
(86) International application number: PCT/JP2008/058017
(87) International publication number: WO 2008/136380

(57) **Abstract**

An object of the present invention is to provide a dry coating method which can coat a tablet in a simple manner and with high efficiency, and therefore can produce a tablet having good appearance and hardly causing breaking/cracking. The present invention provides a process for producing a tablet coated with a coating agent comprising a meltable substance, comprising the steps of: heating a plain tablet to a temperature equal to or higher than a melting point of the meltable substance; and contacting the plain tablet with the coating agent to coat the plain tablet.

## Description

### Technical Field

The present invention relates to a novel process for producing a tablet used as a pharmaceutical product, health food, or the like.

### Background Art

In the fields of pharmaceutical products or health foods, it is preferred to provide a product in a form of tablet for its easiness of administration. Generally, a tablet is provided as a film-coated tablet or a sugar-coated tablet to ensure stability of an active ingredient, to attain good appearance, or to achieve even easier administration with addition of sweet flavor.
However, in preparation of either form of tablet, a plain tablet (an uncoated tablet) is coated with a coating agent which has been dissolved or suspended in a solvent in advance. Thus, the above-described coating could not be applied to a medicinal agent unstable to water or a solvent.
In this circumstance a dry coating method was developed in which a coating agent was applied to a tablet as it was in a solid form, not in a liquid form.

As a previously reported dry coating method for a tablet, for example, a method using a tableting machine for compression coated tablets (tablet within a tablet, dry coated tablet) is known. The method is characterized by compression-coating a core tablet with powder containing a fatty, waxy substance or a thermoplastic substance which melts or softens at 40°C or higher, and further heating a coated tablet (Patent Document 1). However, the above-described method requires a special tableting machine for compression coated tablets. Also, disintegration time tends to become longer because a relatively large volume of a coating agent needs to be used to completely coat a tablet so as to prevent exposure of a core tablet.

Another dry coating method has been reported in which a plain tablet is spray-coated with a finely powdered enteric polymer such as hydroxypropylmethylcellulose phthalate, while the tablet is sprayed with a plasticizing liquid such as triethyl citrate (Patent Document 2). However, a film of the enteric polymer formed by the above-described dry coating method is aimed to prevent water in a sugar-coating suspension from entering the center core tablet and it is not intended to be used as the outermost layer of a finished product. It is necessary to form a sugar-coating layer over the film in order to attain properties such as an appearance and resistance to breaking or cracking necessary for a tablet.

Furthermore, a dry coating method used for a granule having an average particle size of approximately from 50 to 2000 µm, preferably approximately from 100 to 1500 µm, is described in Japanese Patent Laid-Open No. 4-290817 (Patent Document 3). In this method a granule preheated to 65°C which contains a principal agent is contacted and coated with a powder mixture containing an enteric substance and a meltable substance, followed by cooling to the melting point of the meltable substance or lower. In this document, examples of the meltable substance include higher fatty acids, higher aliphatic alcohols, higher fatty acid esters, and hydroxy higher fatty acid esters. It is further described in paragraph 14 of the document that these meltable substances can be used concomitantly with a water-soluble meltable substance such as polyethylene glycol.

However, a dry-coating technique which is able to coat a tablet, which has a larger surface area as compared to a granule, giving it good appearance in a simple manner with high efficiency, while forming a surface hardly causing breaking or cracking has not been found up until now.

Patent Document 1: Japanese Patent Publication No. 39-17168
Patent Document 2: Japanese Patent Laid-Open No. 2002-370971
Patent Document 3: Japanese-Patent Laid-Open No. 4-290817

### Disclosure of the Invention

### Problems to be Solved by the Invention

In view of the above, an object of the present invention is to provide a dry coating method which can coat a tablet in a simple manner and with high efficiency, and therefore can produce a tablet having good appearance and hardly causing breaking or cracking.

### Means for Solving the Problems

Through extensive and research, the present inventors completed the present invention upon finding that the aforementioned problem can be solved when the following constructions are provided.
Specifically, the present invention relates to the following;
[1] a process for producing a tablet coated with a coating agent comprising a meltable substance, comprising the steps of:
   heating a plain tablet to a temperature equal to or higher than a melting point of the meltable substance; and
   contacting the plain tablet with the coating agent to coat the plain tablet.
[2] the process according to item [1], wherein a content of the meltable substance in the coating agent is from 10% to 60%.
[3] the process according to item [1] or [2], wherein the melting point of the meltable substance is from 30°C to 100°C.
[4] the process according to any one of items [1] to [3], wherein the meltable substance is any one or more substance selected from the group consisting of a higher saturated alcohol having a carbon number of 14 or more, a higher saturated fatty acid having a carbon number of 10 or more, a waxy substance, and a polymer substance.
[5] the process according to item [4], wherein the higher saturated alcohol is any one or more alcohols selected from the group consisting of myristyl alcohol, cetyl alcohol, and stearyl alcohol.
[6] the process according to item [4], wherein the higher saturated fatty acid is any one ore more fatty acids selected from the group consisting of capric acid, lauric acid, myristic acid, palmitic acid, stearic acid, and arachidic acid.
[7] the process according to item [4], wherein the waxy substance is any one or more substances selected from the group consisting of bees wax, whale wax, Japan wax, carnauba wax, cacao butter, beef tallow, lard, lanolin, Vaseline, and paraffin.
[8] the process according to item [4], wherein the polymer substance is polyethylene glycol.
[9] the process according to item [8], wherein the polyethylene glycol has an average molecular weight of from 1000 to 50000.
[10] the process according to any one of items [1] to [9], wherein the coating agent comprises any one or more agents selected from the group consisting of a sugar, an inorganic substance, celluloses, and an acrylic acid-based polymer.
[11] the process according to item [10], wherein the sugar is any one or more sugars selected from the group consisting of sucrose, lactose, mannitol, maltitol, erythritol, and xylitol.
[12] the process according to item [10], wherein the inorganic substance is any one or more substances selected from the group consisting of talc, calcium hydrogen phosphate, titanium oxide, magnesium stearate, and calcium carbonate.
[13] the process according to item [10], wherein the celluloses are any one or more celluloses selected from the group consisting of hydroxyethylcellulose, hydroxypropylcellulose, hydroxypropylmethylcellulose, and crystalline cellulose.
[14] the process according to item [10], wherein the acrylic acid-based polymer is any one or more polymers selected from the group consisting of methacrylate copolymer, methacrylate/butyl acrylate copolymer, methacrylate/ethyl acrylate copolymer, ethyl acrylate/methyl methacrylate copolymer, and aminoalkyl methacrylate copolymer.
[15] the process according to any one of items [1] to [14], wherein further comprising, subsequent to the step of coating the plain tablet, cooling the tablet at the temperature equal to or lower than the melting point of the meltable substance while rotating the tablet.
[16] a tablet produced by the process according to any one of items [1] to [15].

### Advantages of the Invention

The process for producing the tablet according to the present invention enables coating of a tablet in a simple manner in a short period of time, the coating of the tablet thus produced has beautiful appearance comparable to that of a sugar-coated tablet and favorable physical properties of, for example, disintegration and resistance to a drop impact. Further, a production cost can be reduced according to the process of the present invention. Furthermore, the process enables thin and uniform coating; therefore, an effect of masking a unique property and a taste of the plain tablet can be obtained, while the tablet is prevented to become larger in size and calories from the coating agent can be suppressed. Moreover, the process enables coating of a tablet containing an ingredient which decomposes when coexisting with water or a solvent because the process does not require a solvent during a process of production.

### Best Mode for Carrying Out the Invention

Preferred embodiments of the present invention are described in detail hereinbelow.
In the process for producing a coated tablet according to the present invention, a plain tablet produced in a conventional manner is heated to a temperature equal to or higher than the melting point of a meltable substance contained in a coating agent. For example, a large quantity of plain tablet can be uniformly heated by pouring the plain tablet into a coating pan or an automated coating machine and heating the tablet while rotating the pan.

Next, the heated plain tablet is contacted with the coating agent containing the meltable substance for coating. For example, this step is conducted by pouring the coating agent into the coating machine either while the pan containing the plain tablet is rotated or while rotation is interrupted, then further rotating the pan. The meltable substance contained in the coating agent melts when the heated plain tablet contacts with the coating agent, and the coating agent favorably attaches to the surface area of the plain tablet. At this point, a uniformly coated tablet with no unevenness can be obtained by conducting the coating in a gradual manner by changing the coating agent normally in 2 to 50 portions, preferably in 3 to 30 portions, more preferably in 5 to 15 portions.

Subsequently, a tablet having a beautiful appearance with an increased surface luster is obtained by cooling the tablet until the temperature thereof is lowered to approximately equal to the room temperature, while rotating the pan of the coating machine.

The meltable substance used in the present invention means a substance which is solid at normal temperature and has a melting point at room temperature or higher. The melting point of the meltable substance is preferably from 30°C to 100°C, more preferably from 50°C to 70°C. Examples of the meltable substance include, but are not limited to, a higher saturated alcohol having a carbon number of 14 or more, a higher saturated fatty acid having a carbon number of 10 or more, a waxy substance, a polymer substance, and the like.

Examples of the higher saturated alcohol include myristyl alcohol, cetyl alcohol, stearyl alcohol, and the like.

Examples of the higher fatty acid include capric acid, lauric acid, myristic acid, palmitic acid, stearic acid, arachidic acid, and the like.

Examples of the waxy substance include bees wax, whale wax, Japan wax, carnauba wax, cacao butter, beef tallow, lard, lanolin, vaseline, paraffin, and the like.

Examples of the polymer substance include polyethylene glycol, which preferably has an average molecular weight of from 1000 to 50000, more preferably from 4000 to approximately 20000, in view of its melting point.

The coating agent of the present invention can contain only one kind or more than two kinds of the meltable substance as described above.

The content of the meltable substance in the coating agent is preferably from 10% to 60%, more preferably from 10% to 50%.

Plural kinds of coating agent having different contents of the meltable substance can be prepared and contacted with the plain tablet in a sequential manner.

Various kinds of substance can be added to the coating agent used in the present invention in addition to the aforementioned meltable substance as long as they can be employed as a coating agent of a tablet. A substance to be added to the coating agent can be sugars such as sucrose, lactose, mannitol, erythritol, maltitol, xylitol, inorganic substances such as talc, calcium hydrogen phosphate, titanium oxide, magnesium stearate, calcium carbonate, celluloses such as hydroxyethylcellulose, hydroxypropylcellulose, hydroxypropylmethylcellulose, crystalline cellulose, acryl acid-based polymers such as methacrylate copolymer, methacrylate/butyl acrylate copolymer, methacrylate/ethyl acrylate copolymer, ethyl acrylate/methyl methacrylate copolymer, aminoalkyl methacrylate copolymer, pigments, and the like.

The coating agent is preferably in a form of powder when poured into the pan of the coating machine. The coating powder can be produced by a conventional manner using the aforementioned ingredient. A uniform coating powder can be produced after appropriately mixing plural kinds of the aforementioned ingredient. Alternatively, two or more kinds of different coating powder, each containing any of the aforementioned ingredients, can be produced in advance and poured into the pan simultaneously or sequentially. When using two or more kinds of coating powder, the content of the meltable substance accounts for preferably 10% to 60% based on the total weight of all the coating powder poured into the pan. Plural kinds of coating powder containing the meltable substance at various rates can be prepared and poured into the pan in a sequential manner.

A plain tablet used in the present invention can be any one produced by a known method, and no particular limitation is imposed thereon. Generally, an appropriate additive is mixed to the plain tablet in addition to an active ingredient upon tableting.

Examples of the active ingredient include an antipyretic analgesic, an antiphlogistic, an antitussive, an antiemetic, an antidepressant, an anticholinergic, an antidementia agent, an antimigraine agent, a psychotropic, a hypnotic, an antidinic, an antispasmodic, an antitumor agent, a muscle relaxant, an antiarrhythmic agent, an antiarteriosclerotic agent, a hypotensive agent, a blood circulation promoter, a cardiotonic, a diuretic, an antiasthmatic agent, an antiallergic agent, an antidiabetic agent, an antihistamine agent, an antirheumatic agent, an immunomodulator, an antiparasitic, an antibacterial agent, an antifungal agent, an antiviral agent, an expectorant, a hypolipidemic, an antiosteoporotic agent, a cholagogue, a vitamin preparation, and the like.

The additive can be an agent which is generally used for a medicine such as an excipient, a binder, a lubricant, a disintegrator, a colorant, a flavoring agent, an emulsifier, a surfactant, a solubilizing agent, a suspending agent, an isotonic agent, a buffer, an antiseptic, an antioxidant, a stabilizer, an absorption promoter. The additives can be used in combination appropriately as desired.

Examples of the excipient include lactose, sucrose, glucose, corn starch, mannitol, sorbitol, starch, pregelatinized starch, dextrin, crystalline cellulose, light anhydrous silicic acid, aluminum silicate, calcium silicate, magnesium aluminometasilicate, calcium hydrogen phosphate, and the like.

Examples of the binder include polyvinyl alcohol, methylcellulose, ethylcellulose, gum arabic, tragacanth, gelatin, shellac, hydroxypropylmethylcellulose, hydroxypropylcellulose, sodium carboxymethylcellulose, polyvinyl pyrrolidone, macrogol, and the like.

Examples of the lubricant include magnesium stearate, calcium stearate, sodium stearyl fumarate, talc, polyethylene glycol, colloidal silica, and the like.

Examples of the disintegrator include crystalline cellulose, agar, gelatin, calcium carbonate, sodium bicarbonate, calcium citrate, dextrin, pectin, hydroxypropylcellulose with a low degree of substitution, carboxymethylcellulose, carboxymethylcellulose calcium, croscarmellose sodium, carboxymethyl starch, carboxymethyl starch sodium, and the like.
Examples of the colorant include an agent which is allowed to be added to a pharmaceutical product, for example, ferrous oxide, ferric oxide, ferrous oxide yellow, ferric oxide yellow, carmine, caramel, β-carotene, titanium oxide, talc, riboflavin sodium phosphate, yellow aluminum lake, and the like.

Examples of the flavoring agent include cocoa powder, peppermint camphor, fragrant powder, peppermint oil, menthol, borneol, cinnamon powder, and the like.

Examples of the emulsifier or the surfactant include stearyl triethanolamine, sodium lauryl sulfate, laurylaminopropionic acid, lecithin, glyceryl monostearate, sucrose fatty acid ester, glycerin fatty acid ester, and the like.

Examples of the solubilizing agent include polyethylene glycol, propylene glycol, benzyl benzoate, ethanol, cholesterol, triethanolamine, sodium carbonate, sodium citrate, polysorbate 80, nicotinamide, and the like.

Examples of the suspending agent include, in addition to the aforementioned surfactant, a hydrophilic polymer, for example, polyvinyl alcohol, polyvinylpyrrolidone, methylcellulose, hydroxymethylcellulose, hydroxyethylcellulose, hydroxypropylcellulose, and the like.

Examples of the isotonic agent include glucose, sodium chloride, mannitol, sorbitol, and the like.

Examples of the buffer include a buffer solution of, for example, phosphate, acetate, carbonate, citrate, and the like.

Examples of the antiseptic include methylparaben, propylparaben, chlorobutanol, benzyl alcohol, phenethyl alcohol, dehydroacetic acid, sorbic acid, and the like.

Examples of the antioxidant include sulfite, ascorbic acid, α-tocopherol, and the like.

Examples of the stabilizer include ascorbic acid, sodium edetate, erythorbic acid, tocopherol, and the like.

Examples of the absorption promoter include isopropyl myristate, tocopherol, calciferol, and the like.

### Examples

The present invention is described further in detail with examples hereinbelow; however, it should not be construed that the present invention is limited to these examples.

### [Example 1]

Using a tumbler mixer (model: WP-50, Tokuju Corporation), 6000 g of lactose, 3000 g of crystalline cellulose (CEOLUS 101, Asahi Kasei Corporation), 980 g of corn starch and 20 g of calcium stearate were mixed. The obtained powder mixture was then tableted using a rotary tablet press (model: AP-15SS, Hata Iron Works Co., Ltd.) with a mortar having a diameter of 7.5 mm so that each tablet had a weight of 160 mg, thereby plain tablets were obtained.
Then, polyethylene glycol 6000 (MACROGOL 6000, Sanyo Chemical Industries, Ltd., which will be hereinafter expressed as PEG 6000) and talc were mixed at a weight ratio of 5:5, 4:6, 3:7, 2:8, and 1:9 to give coating powder.
Coating of 7000 g of the plain tablets was conducted using a coating machine (model: Hicoater HC-MULTI, Freund Corporation) with the coating powder. The tablets were poured into the pan of the coating machine, followed by heating (inlet air temperature: 80°C). It was controlled so that the temperature of the tablets was 62°C to 65°C as the melting point of PEG 6000 was 60°C to 62°C.
The coating powder was then poured into the coating machine. The five kinds of coating powder mixtures obtained as above were sequentially poured into the pan, 140 g each, in descending order of the content of PEG 6000. After 140 g of the coating powder having a mixing ratio of 1:9 was poured, coating was continued with the coating powder until the amount of coating reached 20% by weight of the plain tablets. Subsequently, the tablets were cooled until the temperature thereof was lowered to approximately equal to the room temperature, while rotation of the pan of the coating machine was continued.

### [Example 2]

In a similar manner to Example 1, 7000 g of the plain tablets were produced and similarly heated using the coating machine (model: HC-MULTI, Freund Corporation). The coating machine was charged with 70 g of PEG 6000, and 70 g of talc was poured thereto after PEG melted. Coating was then conducted by pouring PEG and talc alternately so that the weight ratio of PEG to talc was 4:6, 3:7, and 2:8, where the total amount of input was 140 g each time. Thereafter, PEG and talc were alternately poured so that the weight ratio of PEG to talc was 1:9, where the total amount of input was 140 g, and coating was continued until the amount of coating reached 20% by weight of the plain tablets. The tablets were then cooled.

### [Comparative Example 1]

### Film-coated tablet

In 13500 g of purified water, 840 g of hydroxypropylmethylcellulose and 180 g of PEG 6000 were dissolved. Then, 240 g of talc and 240 g of titanium oxide were uniformly dispersed therein using a homomixer, thereby a film-coating suspension was prepared. The film-coating suspension thus prepared was sprayed to 7000 g of the plain tablets produced in a similar manner to Example 1 in the Hicoater, thereby film-coated tablets each having 20 mg of film layer were obtained.

### [Comparative Example 2]

### Sugar-coated tablet

In 3520 g of purified water, 800 g of gum arabic powder, 2540 g of precipitated calcium carbonate, 2020 g of talc, 240 g of titanium oxide, and 6880 g of purified sucrose were dissolved or dispersed, thereby subcoating suspension was prepared. In 3580 g of purified water, 420 g of titanium oxide, 100 g of gum arabic, and 6700 g of purified sucrose were dissolved or dispersed, thereby color coating suspension was obtained. In the Hicoater 7000 g of the plain tablets produced in a similar manner to Example 1 were coated with the sugar-coating liquid thus prepared, thereby a sugar-coated tablets each having 85 mg of a subcoating layer and 35 mg of a color coating layer were obtained.

### <Test Example 1>

The dry-coated tablets obtained in Examples 1 and 2, the film-coated tablets obtained in Comparative Example 1, and the sugar-coated tablets obtained in Comparative Example 2 were subjected to visual observation for evaluation of luster and uniformity of the coating. Further, time required for coating with the coating machine and disintegration time were measured, where disintegration time was measured following the disintegration testing method described in Japanese Pharmacopoeia, the 15^{th} edition. The results are shown in Table 1.

**[Table 1]**

| | Ex. 1 | Ex. 2 | Com. Ex. 1 (film-coated tablet) | Com. Ex. 2 (sugar-coated tablet) |
|---|---|---|---|---|
| Luster | Ⓞ | ○ | ○ | Ⓞ |
| Uniformity | Ⓞ | ○ | Ⓞ | ○ |
| Coating time | 1 hour | 1 hour | 4 hours | 7 hours |
| Disintegration time | 4-6 minutes | 5-6 minutes | 12-14 minutes | 17-22 minutes |

Evaluation criteria for luster
   Ⓞ: have excellent luster, ○: have luster, Δ: have little luster, ×: have no luster
Evaluation criteria for uniformity
   Ⓞ: uniform, ○: faintly uneven, Δ: slightly uneven, ×: uneven

As shown in Table 1, the tablets produced in Examples 1 and 2 could be produced in a much shorter time compared to the film-coated tablet and the sugar-coated tablet, while attaining an equivalent quality.

### [Example 3]

Using a tumbler mixer, 1000 g of caffeine, 5000 g of lactose, 3000 g of crystalline cellulose (CEOLUS 101), 980 g of corn starch, and 20 g of calcium stearate were mixed. The obtained powder mixture was then tableted using a rotary tablet press with a mortar having a diameter of 7.5 mm so that each tablet had a weight of 160 mg, thereby plain tablets were obtained.
In a similar manner to Example 1, plural kinds of coating powder containing PEG 6000 and talc at various weight ratios were then produced and coating of the plain tablets were conducted with the coating powder thus produced, followed by cooling.

### <Test Example 2>

Ten experienced panelists evaluated whether or not the bitterness of caffeine was tasted by holding the coated tablet and the plain tablet produced by the method of Example 3 in the mouth, each separately. As a result, all of ten panelists tasted the strong bitterness with the plain tablet, whereas none of them tasted the bitterness with the coated tablet of the present invention.

### [Example 4]

Using a tumbler mixer, 1260 g of riboflavin phosphate, 4740 g of lactose, 3000 g of crystalline cellulose (CEOLUS 101), 980 g of corn starch, and 20 g of calcium stearate were mixed. The obtained powder mixture was then tableted using a rotary tablet press with a mortar having a diameter of 7.5 mm so that each tablet had a weight of 160 mg, thereby plain tablets were obtained.
Titanium oxide and talc were then mixed at weight ratio of 1:1, and further mixed and pulverized using a hammermill. PEG 6000 and the pulverized powder thus obtained were then mixed at weight ratios of 5:5, 4:6, 3:7, 2:8, and 1:9 to obtained coating powder.
Thereafter, coating of the tablet was conducted, followed by cooling, in a similar manner to Example 1.

### [Example 5]

Using a tumbler mixer, 1000 g of fursultiamine hydrochloride, 5000 g of lactose, 3000 g of crystalline cellulose (CEOLUS 101), 980 g of corn starch, and 20 g of calcium stearate were mixed. The obtained powder mixture was then tableted using a rotary tablet press with a mortar having a diameter of 7.5 mm so that each tablet had a weight of 160 mg, thereby plain tablets were obtained.
PEG 6000 and talc were mixed at weight ratios of 5:5, 4:6, 3:7, 2:8, and 1:9 to obtain coating powder. Then, coating of the plain tablets was conducted, followed by cooling, in a similar manner to Example 1.

### [Example 6]

PEG 6000 and lactose were mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured to conduct coating of the plain tablets, followed by cooling.

### [Example 7]

PEG 6000 and mannitol were mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured to conduct coating of the plain tablets, followed by cooling.

### [Example 8]

PEG 6000 and erythritol were mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured to conduct coating of the plain tablets, followed by cooling.

### [Example 9]

PEG 6000 and maltitol were mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured to conduct coating of the plain tablets, followed by cooling.

### [Example 10]

PEG 6000 and calcium hydrogen phosphate were mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured to conduct coating of the plain tablets, followed by cooling.

### [Example 11]

Crystalline cellulose and talc were mixed at weight ratio of 1:1, and further mixed and pulverized using a hammermill. PEG 6000 and the pulverized powder thus obtained were then mixed at weight ratios of 5:5, 4:6, 3:7, 2:8, and 1:9 to obtain coating powder.
In a similar manner to Example 1, 7000 g of plain tablets was produced and heated in the coating machine (HC-MULTI, Freund Corporation), to which the coating powder thus obtained was poured for coating of the plain tablets, followed by cooling.

### <Test Example 3>

The tablets obtained in Examples 1 through 11 were subjected to visual observation for evaluation of roundness, luster, and uniformity of the coating. Further, disintegration time was measured following the disintegration testing method described in Japanese Pharmacopoeia, the 15^{th} edition. The results are shown in Table 2.

**[Table 2]**

| Ex. | 1 | 2 | 3 | 4 | 5 | 6 | 7 | 8 | 9 | 10 | 11 |
|---|---|---|---|---|---|---|---|---|---|---|---|
| Roundness | Ⓞ | Ⓞ | Ⓞ | Ⓞ | ⊚ | ○ | ○ | ○ | ○ | ○ | ○ |
| Luster | Ⓞ | ○ | Ⓞ | ○ | ○ | ○ | ○ | ○ | ○ | ○ | ○ |
| Uniformity | Ⓞ | ○ | Ⓞ | Ⓞ | Ⓞ | ○ | ○ | ○ | ○ | ○ | ○ |
| Disintegration (minute) | 4-6 | 5-6 | - | - | - | 4-7 | 3-6 | 3-6 | 3-5 | 4-5 | 5-6 |

| | | | | | | | | | | | |
|---|---|---|---|---|---|---|---|---|---|---|---|
| - : No data | | | | | | | | | | | |

Evaluation criteria for roundness
   Ⓞ: smoothly round, ○: round, Δ: slightly edgy, ×: edgy
Evaluation criteria for luster
   Ⓞ: have excellent luster, ○: have luster, Δ: have little luster, ×: have no luster
Evaluation criteria for uniformity
   Ⓞ: uniform, ○: faintly uneven, Δ: slightly uneven, × : uneven

As shown in Table 2, any of the tablets obtained in Examples 1 through 11 was superior in appearance and disintegration property, and had a uniform coating layer. Furthermore, the tablets had superior resistance to a drop impact.

### [Comparative Example 3]

PEG 6000 was heated to approximately 70°C and melted, to which pulverized sucrose was added. The mixture was then uniformly mixed, while it was kept warm approximately at 60°C. Subsequently the mixture was left to cool, and when it was slightly hardened, it was sieved through mesh 16 for granulation. A plain tablet having a diameter of 6.5 mm and a weight of 90 mg was provided as a core tablet, and tableting was conducted using a single tablet press (model: N-30E, Okada Seiko Co., Ltd.) so as to cover the core tablet with the powder mixture thus obtained, thereby a compression coated tablet having a diameter of 10 mm and a weight of 490 mg was produced. The tablets thus prepared were poured into the pan of the coating machine and heated for 20 minutes to 60°C while rotating, and then cooled with continuous rotation.

### [Comparative Example 4]

PEG 6000 was heated to approximately 70°C and melted, to which talc was added. The mixture was then uniformly mixed, while it was kept warm approximately at 60°C. Subsequently the mixture was left to cool, and when it was slightly hardened, it was sieved through mesh 16 for granulation. A plain tablet having a diameter of 6.5 mm and a weight of 90 mg was provided as a core tablet, and tableting was conducted using a single tablet press so as to cover the core tablet with the powder mixture thus obtained, thereby a compression coated tablet having a diameter of 10 mm and a weight of 540 mg were produced. The tablets thus obtained were poured into the pan of the coating machine and heated for 20 minutes to 60°C while rotating, and then cooled with continuous rotation.

The tablets obtained in Comparative Examples 3 and 4 were measured for hardness using the Kiya hardness tester and for disintegration time following the disintegration testing method described in Japanese Pharmacopoeia, the 15^{th} edition. The results are shown in Table 3.

**[Table 3]**

| Test item | Plain tablet | Com. Ex. 3 | | Com. Ex. 4 | |
|---|---|---|---|---|---|
| | | After tableting | After coating | After tableting | After coating |
| Amount of coating (mg) | - | 400 | 400 | 450 | 450 |
| Hardness (N) | - | 158.4 | 181.4 | 107.0 | 129.2 |
| Disintegration time (minute) | 0.5 or less | 7.0 | 7.7 | 19.3 | 24.0 |

| | | | | | |
|---|---|---|---|---|---|
| -: No data | | | | | |

As shown in Table 3, the compression coated tablets of Comparative Example 4 marked a prominently long disintegration time. Furthermore, the compression coated tablets of Comparative Examples 3 and 4 became larger in size because the amount of the coating agent needed was 4 to 5 times as much as the weight of the plain tablets.

## Claims

1. A process for producing a tablet coated with a coating agent comprising a meltable substance, comprising the steps of:
heating a plain tablet to a temperature equal to or higher than a melting point of the meltable substance; and
contacting the plain tablet with the coating agent to coat the plain tablet.

2. The process according to Claim 1, wherein a content of the meltable substance in the coating agent is from 10% to 60%.

3. The process according to Claim 1 or 2, wherein the melting point of the meltable substance is from 30°C to 100°C.

4. The process according to any one of Claims 1 to 3, wherein the meltable substance is any one or more substance selected from the group consisting of a higher saturated alcohol having a carbon number of 14 or more, a higher saturated fatty acid having a carbon number of 10 or more, a waxy substance, and a polymer substance.

5. The process according to Claim 4, wherein the higher saturated alcohol is any one or more alcohols selected from the group consisting of myristyl alcohol, cetyl alcohol, and stearyl alcohol.

6. The process according to Claim 4, wherein the higher saturated fatty acid is any one ore more fatty acids selected from the group consisting of capric acid, lauric acid, myristic acid, palmitic acid, stearic acid, and arachidic acid.

7. The process according to Claim 4, wherein the waxy substance is any one or more substances selected from the group consisting of bees wax, whale wax, Japan wax, carnauba wax, cacao butter, beef tallow, lard, lanolin, Vaseline, and paraffin.

8. The process according to Claim 4, wherein the polymer substance is polyethylene glycol.

9. The process according to Claim 8, wherein the polyethylene glycol has an average molecular weight of from 1000 to 50000.

10. The process according to any one of Claims 1 to 9, wherein the coating agent comprises any one or more agents selected from the group consisting of a sugar, an inorganic substance, celluloses, and an acrylic acid-based polymer.

11. The process according to Claim 10, wherein the sugar is any one or more sugars selected from the group consisting of sucrose, lactose, mannitol, maltitol, erythritol, and xylitol.

12. The process according to Claim 10, wherein the inorganic substance is any one or more substances selected from the group consisting of talc, calcium hydrogen phosphate, titanium oxide, magnesium stearate, and calcium carbonate.

13. The process according to Claim 10, wherein the celluloses are any one or more celluloses selected from the group consisting of hydroxyethylcellulose, hydroxypropylcellulose, hydroxypropylmethylcellulose, and crystalline cellulose.

14. The process according to Claim 10, wherein the acrylic acid-based polymer is any one or more polymers selected from the group consisting of methacrylate copolymer, methacrylate/butyl acrylate copolymer, methacrylate/ethyl acrylate copolymer, ethyl acrylate/methyl methacrylate copolymer, and aminoalkyl methacrylate copolymer.

15. The process according to any one of Claims 1 to 14, wherein further comprising, subsequent to the step of coating the plain tablet, cooling the tablet at the temperature equal to or lower than the melting point of the meltable substance while rotating the tablet.

16. A tablet produced by the process according to any one of Claims 1 to 15.
